# EUROPÄISCHE PATENTSCHRIFT

(11) **EP 1 627 607 B1**
(45) Veröffentlichungstag und Bekanntmachung des Hinweises auf die Patenterteilung: **29.11.2006**
(21) Anmeldenummer: 04016985.6
(22) Anmeldetag: 19.07.2004
(51) Int. Cl.: A61B 17/16, A61B 17/17, A61F 2/36

(54) **Implantatsystem**
Implant system
Système d'implant

(43) Veröffentlichungstag der Anmeldung: 22.02.2006
(73) Patentinhaber: Zimmer GmbH, 8404 Winterthur (CH)
(72) Erfinder: Nötzli, Hubert, 3097 Liebefeld (CH); Willi, Roland, 8413 Neftenbach (CH)
(74) Vertreter: Manitz, Finsterwald & Partner GbR

(56) Entgegenhaltungen:
- FR-A- 2 691 897
- US-A- 5 169 402
- US-A- 5 755 811

## Beschreibung

Die Erfindung betrifft ein Implantatsystem mit zumindest einem zementfrei verankerbaren Schaft einer Prothese, insbesondere einer Hüftgelenkprothese, und einem Instrumentensatz zur Vorbereitung des Knochens, in dem der Schaft zu verankern ist, gemäß Oberbegriff des Anspruchs 1.

Ein zementfrei verankerbarer Schaft einer Hüftgelenkprothese ist aus EP 0 135 755 A1 bekannt. Dieser Prothesenschaft ist gerade ausgeführt, besitzt einen trapezförmigen Querschnitt und weist im proximalen Bereich rillenartige Vertiefungen auf. Die hierdurch gebildete Rippenstruktur soll die Aufnahme von Torsionskräften im proximalen Schaftbereich verbessern. Außerdem wird auf US 5,169,402 verwiesen.

Problematisch beim Einsetzen von geraden Schäften ist, dass hierdurch der Trochanter major des Oberschenkelknochens beeinträchtigt werden kann. Bei Verwendung von gekrümmten Schäften dagegen kann der große Trochanter zwar erhalten werden. Das Einsetzen eines gekrümmten Schaftes ist jedoch wiederum problematisch, wenn die Schäfte Rippenstrukturen aufweisen sollen, wie sie vorstehend erwähnt wurden.

Ein entsprechender Schaft ist z.B. aus FR-A-2691897 bekannt.

Der Grund hierfür ist, dass der Schaft beim Einschlagen auf mehreren verschiedenen Bewegungsbahnen in eine vorgegebene Endlage im Knochen gelangen kann. Dies ist unproblematisch, solange der Schaft keine Rippenstrukturen aufweist. Ist der Schaft aber mit Rippenstrukturen versehen, so besteht die Gefahr, dass sich die Rippen eine eigene Spur in den Knochen schneiden, die den Schaft auf eine falsche Bahn geraten lässt, auf welcher der Schaft zumindest nicht mehr in der gewünschten Weise in die Endlage getrieben werden kann.

Aufgrund dieser beim Einsetzen entstehenden Probleme haben mit Rippen versehene gekrümmte Schäfte nicht die Verbreitung gefunden, die wegen der an sich vorhandenen Vorteile derartiger Schäfte zu erwarten gewesen wäre.

Aufgabe der Erfindung ist es, ein Implantatsystem zu schaffen, mit dem auch gekrümmte Prothesenschäfte auf einfache und zuverlässige Weise sicher im Knochen verankert werden können.

Die Lösung dieser Aufgabe erfolgt durch die Merkmale des Anspruchs 1 und insbesondere dadurch, dass der Schaft einen bogenförmigen Grundkörper aufweist und der Instrumentensatz ein entsprechend der Bogenform des Schaftes geformtes erstes Instrument umfasst, mit dem im Knochen eine zur Aufnahme des Grundkörpers dienende Vertiefung herstellbar ist, und wobei der Schaft im proximalen Bereich mit Rippen versehen ist und der Instrumentensatz einen entsprechend der Bogenform des Schaftes geformten Führungskörper für ein zweites Instrument umfasst, mit dem bei in der Vertiefung befindlichem Führungskörper zur Festlegung einer Bahn für die Schaftrippen dienende Strukturen im Knochen herstellbar sind.

Erfindungsgemäß ist zusätzlich zu dem zur Herstellung der Vertiefung für den Grundkörper dienenden Instrument, insbesondere einer Raspel, ein weiteres Instrument vorgesehen, mit dem der Knochen gezielt zur Führung der Schaftrippen vorbereitet werden kann. Dabei zeichnet sich die Erfindung dadurch aus, dass für die mittels des zweiten Instruments durchzuführende Bearbeitung die zuvor mittels des ersten Instruments hergestellte Vertiefung genutzt wird, indem ein ebenfalls entsprechend dem Grundkörper des Schaftes geformter Führungskörper zum Einsatz kommt, der in die Vertiefung eingesetzt wird und dann als Führung für das zweite Instrument dient. Mittels des definiert geführten zweiten Instruments wird anschließend eine Bahn für die Schaftrippen und damit für den Schaft insgesamt festgelegt, der im Anschluss an die mittels des zweiten Instruments erfolgende Knochenbearbeitung eingeschlagen wird.

Indem als Referenz für die Bearbeitungsbewegung des zweiten Instruments ein hinsichtlich der Bogenform dem Schaft entsprechender Führungskörper dient, kann erfindungsgemäß sichergestellt werden, dass für die Schaftrippen eine Spur hergestellt und damit eine Bahn vorgegeben wird, die auf die Bogenform des Grundkörpers des Schaftes abgestimmt ist. Hierdurch ist gewährleistet, dass der mit den Rippen versehene gekrümmte Schaft in der gewünschten Weise in die zuvor mittels des ersten Instruments vorgegebene Endlage gelangt.

Die Rippen bzw. Tangenten an den Rippen verlaufen entsprechend der Schaftkrümmung unter einem von Null verschiedenen Winkel zur Schaftachse. Dieser Rippenwinkel liegt insbesondere im Bereich von 0° bis 45°, wobei aber auch größere Winkel möglich sind, und wird in Abhängigkeit von der Bogenform des Schaftes gewählt.

Der Führungskörper kann in Form eines separaten Teils vorgesehen sein. Alternativ kann das erste Instrument gleichzeitig als Führungskörper für das zweite Instrument ausgebildet sein. In diesem Fall kann auf einen zusätzlichen Führungskörper verzichtet werden.

Weitere bevorzugte Ausführungsformen der Erfindung sind auch in den abhängigen Ansprüchen, der Beschreibung sowie der Zeichnung angegeben.

So ist vorzugsweise vorgesehen, dass der Schaftquerschnitt rechteckig oder trapezförmig mit ausgeprägten Längskanten ist, welche den Kontakt zur Kortikalis herstellen. Im Weiteren ist bevorzugt vorgesehen, dass die Bogenform des Grundkörpers insbesondere hinsichtlich Krümmung und Länge derart in Bezug auf zwei sich gegenüberliegende Kanten, d.h. eine laterale Kante und eine mediale Kante, gewählt ist, dass sich eine durch mehrere, insbesondere drei, Auflagestellen an der Kortikalis des Knochens eindeutig definierte Endlage des Grundkörpers ergibt. Hierdurch ist sichergestellt, dass sich der jeweilige Grundkörper beim Einsetzen entsprechend seinem Rechteck- oder Trapezquerschnitt selbst zentriert und in der Endlage keine Hohlräume mehr zwischen dem Grundkörper und dem von der Kortikalis gebildeten Knochenbett vorhanden sind, da wegen der keilförmigen Ausführung auch eine Verspannung zwischen den beiden lateralen sowie zwischen den beiden medialen Kanten stattfindet. Insbesondere sind die Krümmung des Grundkörpers und die Länge des Grundkörpers bzw. des Schaftes in einer vorgegebenen Weise aufeinander abgestimmt.

Besonders vorteilhaft ist es, wenn gemäß einer weiteren Ausführung der Erfindung der Verlauf der Schaftrippen auf die Bogenform des Grundkörpers des Schaftes derart abgestimmt ist, dass zwischen gegenüberliegenden lateralen und medialen Kanten eine durch mehrere, insbesondere drei, Auflagestellen an der Kortikalis des Knochens eindeutig definierte Endlage des Grundkörpers erreichbar ist. Dabei ist vorgesehen, dass die Auflagestellen nicht punktförmig, sondern in Form von in Längsrichtung gestreckten Zonen gebildet werden.

Die Rippen des Schaftes können sowohl gerade als auch gekrümmt verlaufen. Dabei ist es nicht zwingend, dass alle Rippen gerade oder alle Rippen gekrümmt ausgeführt sind. Innerhalb einer mehrere Rippen umfassenden Rippenstruktur können sowohl gerade als auch gekrümmte Rippen vorgesehen sein. Prinzipiell ist es auch möglich, auf jeder Seite des Schaftes lediglich eine einzige Rippe vorzusehen. Möglich ist ferner, dass lediglich auf einer Schaftseite eine oder mehrere Rippen vorgesehen sind.

Der Verlauf der Rippen kann insbesondere von deren jeweiliger Länge abhängig gemacht werden. Dabei können eine bestimmte Länge nicht übersteigende Rippen gerade ausgeführt sein, wohingegen eine bestimmte Länge übersteigende Rippen gekrümmt verlaufen können.

Bevorzugt ist weiterhin vorgesehen, dass die Schaftrippen sich ausgehend von einer zumindest näherungsweise gemeinsamen Ebene nach distal erstrecken. Vorzugsweise ist diese gemeinsame Ebene bei eingesetztem Schaft im Bereich einer Resektionsebene des Knochens gelegen.

Wenn sowohl gerade als auch gekrümmte Schaftrippen vorhanden sind, dann ist gemäß einer weiteren Ausführungsform der Erfindung bevorzugt vorgesehen, dass die geraden Schaftrippen einerseits und Tangenten an den gekrümmten Schaftrippen andererseits unter dem gleichen von Null verschiedenen Winkel zur Schaftachse verlaufen.

Insbesondere bei einem relativ langen Schaft kann vorgesehen sein, dass dessen Grundkörper im Bereich seines distalen Endes an seiner lateralen Seite mit einer Abflachung versehen ist. Dies gilt bevorzugt auch für das erste Instrument sowie den Führungskörper. Hierdurch wird das Einhalten einer optimalen Bahn beim Einschlagen des Schaftes bzw. beim Einführen des ersten Instruments und des Führungskörpers erleichtert. Die distale Spitze kann jeweils asymmetrisch gegen medial verschoben sein.

Des Weiteren wird vorgeschlagen, dass das erste Instrument im distalen Endbereich an der anterioren und posterioren Seite jeweils mit einer Abschrägung versehen ist.

Ferner kann vorgesehen sein, dass das distale Ende des ersten Instrumentes in distaler Richtung über einen dem Grundkörper des Schaftes entsprechenden Grundkörper des ersten Instrumentes hinaus vorsteht. Hierdurch ist das erste Instrument im Vergleich zum Schaft mit einer distalen Verlängerung versehen.

Vorzugsweise ist das zweite Instrument, mit dem die zur Festlegung einer Bahn für die Schaftrippen dienenden Strukturen im Knochen hergestellt werden, längs einer durch den Führungskörper vorgegebenen Führungsbahn nach distal bewegbar.

Damit die Schaftrippen beim Einschlagen des Schaftes in einer eigenen, vorgegebenen Spur laufen, sind diese Führungsbahn und die Schaftrippen hinsichtlich ihres Verlaufes aufeinander abgestimmt. Bevorzugt ist die Führungsbahn entsprechend gekrümmt verlaufenden Schaftrippen gekrümmt.

Des Weiteren ist vorzugsweise die am Führungskörper für das zweite Instrument vorgesehene Führung ganz oder bereichsweise als Zwangsführung ausgebildet.

Ferner ist vorzugsweise vorgesehen, dass das zweite Instrument U- oder gabelförmig ausgebildet und mit den freien Enden der U- bzw. Gabelarme voran auf den Führungskörper aufschiebbar ist.

Des Weiteren wird erfindungsgemäß vorgeschlagen, dass das zweite Instrument auf seiner vorderen und/oder hinteren Seite mit einer der Anzahl der Schaftrippen entsprechenden Zahl von Bearbeitungsstegen versehen ist. Die Bearbeitungsstege sind auf die Geometrie der Schaftrippen abgestimmt und bevorzugt jeweils als Meißel oder Raspel ausgebildet.

Mit dem zweiten Instrument kann folglich im Knochen ein zumindest teilweises Negativ des mit den Rippen versehenen Schaftbereiches hergestellt werden, wodurch die Rippen beim Einschlagen des Schaftes von vornherein auf die richtige Bahn gezwungen werden.

Die Bearbeitungsstege können jeweils kürzer und/oder niedriger sein als die entsprechenden Schaftrippen. Die in Abhängigkeit von der Länge der am Führungskörper ausgebildeten Führung für das zweite Instrument hierdurch herstellbare Spurlänge reicht für eine eindeutige Spurbildung im Knochen aus.

Des Weiteren ist bevorzugt vorgesehen, dass alle Bearbeitungsstege zumindest im Wesentlichen die gleiche Länge aufweisen.

Die Erfindung wird im Folgenden beispielhaft unter Bezugnahme auf die Zeichnung beschrieben. Es zeigen:
- Fig. 1: verschiedene Ansichten eines Schaftes gemäß einer Ausführungsform der Erfindung,
- Fig. 2: verschiedene Ansichten eines als Raspel ausgebildeten ersten Werkzeugs gemäß einer Ausführungsform der Erfindung,
- Fig. 3: verschiedene Ansichten eines Führungskörpers sowie eines mit dem Führungskörper koppelbaren, als Meißel oder Raspel ausgebildeten zweiten Werkzeugs gemäß einer Ausführungsform der Erfindung,
- Fig. 4: den Führungskörper und das zweite Werkzeug von Fig. 3 im gekoppelten Zustand in verschiedenen Relativstellungen, und

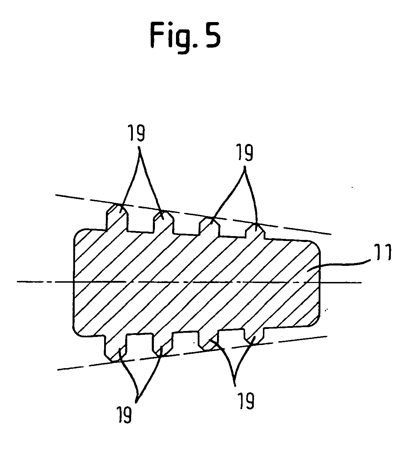

- Fig. 5: in vergrößerter Darstellung einen Querschnitt im gekrümmten proximalen Teil des Schaftes von Fig. 1.

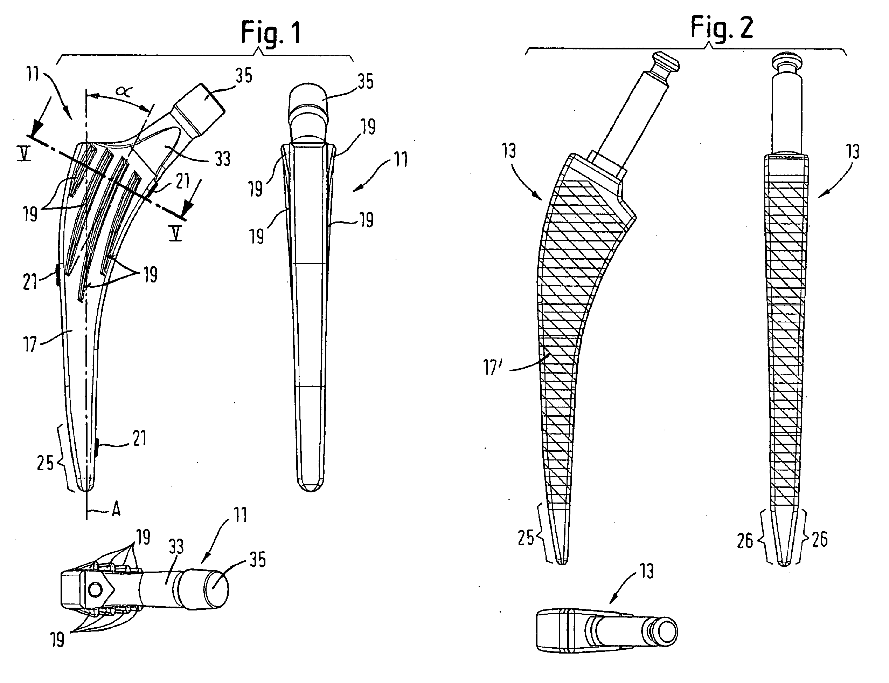

Der in Fig. 1 dargestellte erfindungsgemäße Schaft 11 einer Hüftgelenkprothese weist einen bogenförmig gekrümmten Grundkörper 17, einen Hals 33 sowie einen Kopplungsabschnitt 35 für einen nicht dargestellten Prothesenkopf auf. Dabei ist die Bogenform des Grundkörpers 17 von sich konisch in Richtung der Halsachse öffnender Gestalt.

Der Grundkörper 17 besitzt einen rechteckigen oder trapezförmigen Querschnitt mit ausgeprägten Kanten in seiner Längsrichtung.

Im proximalen Bereich des Grundkörpers 17 sind auf die Vorderseite und die Rückseite des Schaftes 11 jeweils vier parallel verlaufende Rippen 19 von unterschiedlicher Länge aufgesetzt, die sich ausgehend von einer näherungsweise gemeinsamen Ebene nach distal erstrecken. Diese Ebene ist derart gewählt, dass sie bei in den nicht dargestellten Knochen eingesetztem Schaft 11 im Bereich der Resektionsebene des Knochens liegt.

Ausgehend von dieser Ebene verlaufen die Rippen 19 jeweils gerade, wobei sich bei den längeren Rippen 19 jeweils ein leicht gekrümmter Rippenabschnitt mit konstantem Krümmungsradius anschließt, wobei jeweils der proximale gerade Rippenabschnitt mit der Tangente an den gekrümmten Rippenabschnitt zusammenfällt.

Die geraden Rippenabschnitte und damit die Tangenten an den gekrümmten Rippenabschnitten verlaufen unter einem von Null verschiedenen Winkel α zur Schaftachse A.

Die Höhe der Rippen 19 nimmt nach distal ab. Wie insbesondere der unteren Darstellung in Fig. 1 zu entnehmen ist, besitzen die Rippen 19 jeweils im Querschnitt einen rechteckigen Sockel mit einem aufgesetzten Dreieck, d.h. die Rippen 19 laufen nach oben spitz zu.

Die Bogenform des Grundkörpers 17 ist derart gewählt, dass in der gewünschten Endlage des Schaftes 11 im Knochen der Grundkörper 17 mit seinen jeweils gegenüberliegenden lateralen und medialen Kanten an drei Auflagestellen 21 an der das Knochenbett für den Grundkörper 17 bildenden Kortikalis des Knochens aufliegt. Dies gewährleistet, dass sich der Grundkörper 17 stets in der gleichen Endlage im Knochen verkeilt, bei der keine Hohlräume mehr zwischen dem Grundkörper 17 und dem Knochenbett vorhanden sind und sich der Grundkörper 17 beim Einschlagen des Schaftes 11 selbst zentriert.

Im Bereich seines distalen Endes ist der Grundkörper 17 an seiner lateralen Seite mit einer Abflachung 25 versehen. Das Einschlagen des Schaftes 11 entlang der gewünschten Bahn wird hierdurch erleichtert.

Die in Fig. 2 dargestellte, das erste Instrument im Sinne der Erfindung bildende erfindungsgemäße Raspel 13 zeichnet sich dadurch aus, dass sie einen Grundkörper 17' aufweist, der hinsichtlich der Bogenform dem Grundkörper 17 des Schaftes 11 entspricht. Die Raspel 13 dient dazu, die Markhöhle des Knochens bzw. die Kortikalis so vorzubereiten, dass der Schaft 11 durch Einschlagen in der gewünschten Endlage verkeilt werden kann.

Das distale Ende der Raspel 13 steht in distaler Richtung über den Grundkörper 17' vor und bildet eine Spitze, die auf der ventralen und dorsalen Seite jeweils mit einer Abschrägung 26 versehen ist, um der Krümmung des Femurknochens Rechung zu tragen und den Einsatz des Instruments für den linken und den rechten Femur zu ermöglichen. Dabei ist die Spitze der Raspel mehr gegen medial orientiert, um sich während des ganzen Absenkvorganges besser zentrieren zu können.

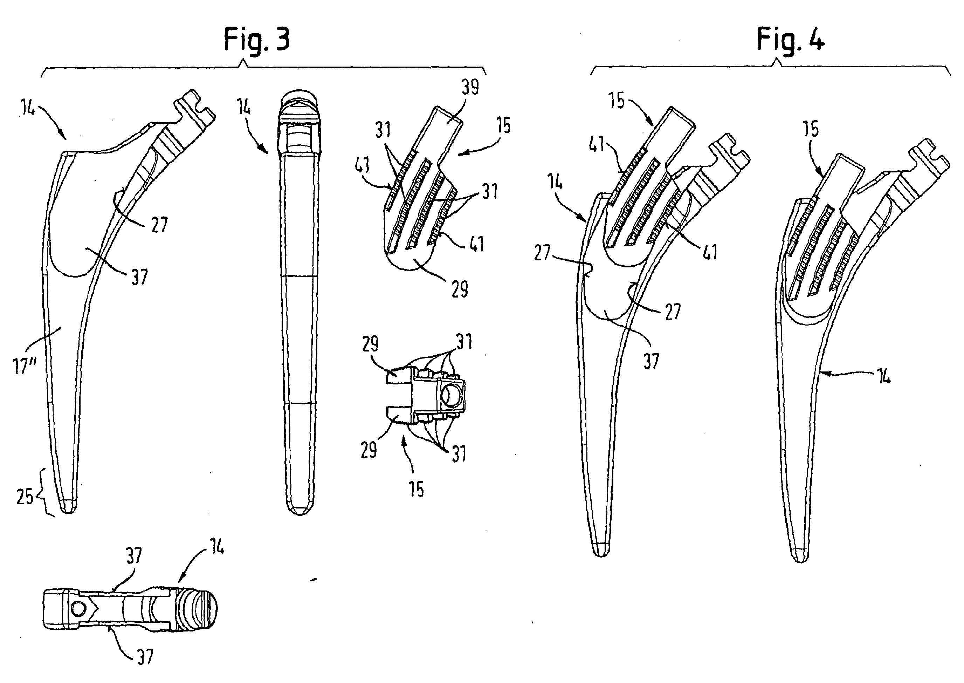

Auch der in Fig. 3 dargestellte erfindungsgemäße Führungskörper 14 ist dem Schaft 11 insofern nachempfunden, als er einen Grundkörper 17" aufweist, der hinsichtlich der Bogenform dem Grundkörper 17 des Schaftes 11 entspricht. Hierdurch sind die Endlagen des Schaftes 11 und des Führungskörpers 14 identisch, so dass das ebenfalls in Fig. 3 dargestellte zweite Instrument 15, auf welches nachstehend näher eingegangen wird, längs des Führungskörpers 14 derart geführt werden kann, dass hierdurch im Knochen Strukturen dort hergestellt werden, wo anschließend der Schaft 11 beim Einschlagen durch seine Rippen 19 geführt wird.

Mittels des Führungskörpers 14 und des am Führungskörper 14 geführten zweiten Instruments 15 kann folglich eine Bahn für die Schaftrippen 19 im Knochen vorgegeben werden, auf welcher der Schaft 11 in der gewünschten Weise mit seinem Grundkörper 17 in die Sollendlage gelangt.

In dem dargestellten Ausführungsbeispiel ist der Führungskörper 14 an seiner Vorderseite und seiner Rückseite mit zurückgesetzten Flachseiten 37 versehen, die durch als Führungsflächen 27 dienende Seitenwände begrenzt sind. Die Krümmung der Führungsflächen 27 ist dabei entsprechend der Krümmung der Schaftrippen 19 derart gewählt, dass beim Einschlagen des Schaftes 11 die Schaftrippen 19 in ihrer eigenen Spur laufen.

Das zweite Instrument 15 ist U- bzw. gabelförmig ausgebildet und umfasst zwei durch einen Kopplungsabschnitt 39 miteinander verbundene Arme 29, wobei der Abstand zwischen den Armen 29 entsprechend der Dicke des Führungskörpers 14 im Bereich der Flachseiten 37 gewählt ist.

Wie Fig. 4 zeigt, ist das zweite Instrument 15 auf den mit den Flachseiten 37 versehenen proximalen Bereich des Führungskörpers 14 aufsteckbar und schlittenartig längs des Führungskörpers 14 verschiebbar, und zwar auf einer Bahn, die durch die mit entsprechenden Gegenflächen 41 des zweiten Instruments 15 (Fig. 3) zusammenwirkenden Führungsflächen 27 vorgegeben ist.

Auf ihren Außenseiten sind die Führungsarme 29 des zweiten Instruments 15 mit jeweils als Meißel oder Raspel ausgebildeten Bearbeitungsstegen 31 versehen. Die Bearbeitungsstege 31 sind entsprechend den Rippen 19 des Schaftes 11 angeordnet, wobei außerdem die Krümmung der Bearbeitungsstege 31 der Krümmung der Schaftrippen 19 entspricht.

Bei einem mittels des erfindungsgemäßen Implantatsystems, wie es vorstehend erläutert wurde, durchführbaren Operationsverfahren wird zunächst mit der Raspel 13 (Fig. 2) eine zur Aufnahme des Schaftes 11 (Fig. 1) dienende Vertiefung im Knochen hergestellt, indem die Markhöhle und die Kortikalis entsprechend bearbeitet werden. Aufgrund der für das erfindungsgemäße Implantatsystem vorgesehenen Bogenform wird der Trochanter major des Oberschenkelknochens nicht beeinträchtigt, denn Vorbereitung und Verankerung erfolgen durch die Resektionsflächen des Knochenhalsansatzes hindurch.

Anschließend wird nach Entfernung der Raspel 13 der Führungskörper 14 (Fig. 3) in die zuvor mittels der Raspel 13 hergestellte Vertiefung eingesetzt. Dabei wird der Führungskörper 14 mit seinem hinsichtlich der Bogenform dem Grundkörper 17 des Schaftes 11 entsprechenden Grundkörper 17" in die gleiche Endlage wie später der Schaft 11 selbst gebracht.

Dann wird das zweite Instrument 15 (Fig. 3) auf die am Führungskörper 14 durch die Führungsflächen 27 gebildete Zwangsführung aufgesteckt. Durch Bewegen des zweiten Instruments 15 entlang der durch die Zwangsführung vorgegebenen Bahn relativ zum Führungskörper 14 werden mittels der Bearbeitungsstege 31 im Knochen Spuren für die Schaftrippen 19 hergestellt.

Nach Entfernung des Führungskörpers 14 und des zweiten Instruments 15 wird schließlich der Schaft 11 eingeschlagen. Dabei laufen die Schaftrippen 19 in den zuvor mittels des zweiten Instruments 15 hergestellten Spuren, die den Schaft 11 auf eine sichere Bahn zwingen, auf welcher der Schaft 11 in der gewünschten Weise durch die Rippen 19 geführt seine zuvor mittels der Raspel 13 vorgegebene Endlage erreicht.

### Bezugszeichenliste

- 11: Schaft
- 13: erstes Instrument, Raspel
- 14: Führungskörper
- 15: zweites Instrument, Meißel
- 17, 17', 17": Grundkörper
- 19: Rippe
- 21: Auflagestelle
- 25: Abflachung
- 26: Abschrägung
- 27: Führungsfläche
- 29: U- bzw. Gabelarm
- 31: Bearbeitungssteg
- 33: Hals
- 35: Kopplungsabschnitt
- 37: Flachseite
- 39: Kopplungsabschnitt
- 41: Gegenfläche

- α: Rippenwinkel
- A: Schaftachse

## Patentansprüche

1. Implantatsystem mit
- zumindest einem zementfrei verankerbaren Schaft (11) einer Prothese, insbesondere einer Hüftgelenkprothese, und
- einem Instrumentensatz (13, 14, 15) zur Vorbereitung des Knochens, in dem der Schaft (11) zu verankern ist,
wobei der Schaft (11) einen bogenförmigen Grundkörper (17) mit Trapez- oder Rechteckquerschnitt aufweist
**dadurch gekennzeichnet,**
**dass** der Instrumentensatz ein entsprechend der Bogenform des Schaftes (11) geformtes erstes Instrument (13) umfasst, mit dem im Knochen eine zur Aufnahme des Grundkörpers (17) dienende Vertiefung herstellbar ist, und
**dass** der Schaft (11) im proximalen Bereich mit Rippen (19) versehen ist und der Instrumentensatz einen entsprechend der Bogenform des Schaftes (11) geformten Führungskörper (14) für ein zweites Instrument (15) umfasst, mit dem bei in der Vertiefung befindlichem Führungskörper (14) zur Festlegung einer Bahn für die Schaftrippen (19) dienende Strukturen im Knochen herstellbar sind.

2. Implantatsystem nach Anspruch 1,
**dadurch gekennzeichnet,**
**dass** der Schaft (11) mit seinem Trapez- oder Rechteckquerschnitt ausgeprägte Kanten in seiner Längsrichtung aufweist.

3. Implantatsystem nach Anspruch 1 oder 2,
**dadurch gekennzeichnet,**
**dass** die Bogenform insbesondere hinsichtlich Krümmung und Länge derart gewählt ist, dass sich für gegenüberliegende Kanten eine durch mehrere, insbesondere drei, Auflagestellen (21) an der Kortikalis des Knochens eindeutig definierte Endlage des Grundkörpers (17) ergibt.

4. Implantatsystem nach einem der vorhergehenden Ansprüche,
**dadurch gekennzeichnet,**
**dass** der Verlauf der Schaftrippen (19) auf die Bogenform derart abgestimmt ist, dass für gegenüberliegende Kanten eine durch mehrere, insbesondere drei, Auflagestellen (21) an der Kortikalis des Knochens eindeutig definierte Endlage des Grundkörpers (17) erreichbar ist.

5. Implantatsystem nach einem der vorhergehenden Ansprüche,
**dadurch gekennzeichnet,**
**dass** zumindest einige Schaftrippen (19) wenigstens bereichsweise gerade ausgeführt sind.

6. Implantatsystem nach einem der vorhergehenden Ansprüche,
**dadurch gekennzeichnet,**
**dass** zumindest einige Schaftrippen (19) wenigstens bereichweise gekrümmt verlaufen, bevorzugt mit einem konstanten Krümmungsradius.

7. Implantatsystem nach einem der vorhergehenden Ansprüche,
**dadurch gekennzeichnet,**
**dass** die Schaftrippen (19) sich ausgehend von einer gemeinsamen Ebene (23) nach distal erstrecken, wobei vorzugsweise bei eingesetztem Schaft (11) die gemeinsame Ebene (23) im Bereich einer Resektionsebene des Knochens gelegen ist.

8. Implantatsystem nach einem der vorhergehenden Ansprüche,
**dadurch gekennzeichnet,**
**dass** die Schaftrippen (19) von unterschiedlicher Länge sind.

9. Implantatsystem nach einem der vorhergehenden Ansprüche,
**dadurch gekennzeichnet,**
**dass** gerade Schaftrippen (19) und Tangenten an gekrümmten Schaftrippen (19) unter dem gleichen von Null verschiedenen Winkel (α) zur Schaftachse (A) verlaufen.

10. Implantatsystem nach einem der vorhergehenden Ansprüche,
**dadurch gekennzeichnet,**
**dass** das distale Ende des ersten Instrumentes (13) in distaler Richtung über einen dem Grundkörper (17) des Schaftes (11) entsprechenden Grundkörper (17') des ersten Instrumentes (13) hinaus vorsteht.

11. Implantatsystem nach einem der vorhergehenden Ansprüche,
**dadurch gekennzeichnet,**
**dass** der Schaft (11), das erste Instrument (13) und der Führungskörper (14) jeweils im Bereich des distalen Endes an der lateralen Seite mit einer Abflachung (25) versehen sind, wobei die distale Spitze asymmetrisch gegen medial verschoben ist.

12. Implantatsystem nach einem der vorhergehenden Ansprüche,
**dadurch gekennzeichnet,**
**dass** das erste Instrument (13) im distalen Endbereich an der anterioren und posterioren Seite jeweils mit einer Abschrägung (26) versehen ist.

13. Implantatsystem nach einem der vorhergehenden Ansprüche,
**dadurch gekennzeichnet,**
**dass** das zweite Instrument (15) längs einer durch den Führungskörper (14) vorgegebenen Führungsbahn nach distal bewegbar ist.

14. Implantatsystem nach Anspruch 13,
**dadurch gekennzeichnet,**
**dass** die Führungsbahn entsprechend gekrümmt verlaufenden Schaftrippen (19) gekrümmt ist.

15. Implantatsystem nach einem der vorhergehenden Ansprüche,
**dadurch gekennzeichnet,**
**dass** das zweite Instrument (15) zumindest bereichsweise am Führungskörper (14) zwangsgeführt ist.

16. Implantatsystem nach einem der vorhergehenden Ansprüche,
**dadurch gekennzeichnet,**
**dass** das zweite Instrument (15) U- oder gabelförmig ausgebildet und mit den freien Enden der U- bzw. Gabelarme (29) voran auf den Führungskörper (14) aufschiebbar ist.

17. Implantatsystem nach einem der vorhergehenden Ansprüche,
**dadurch gekennzeichnet,**
**dass** das zweite Instrument (15) auf seiner vorderen und/oder hinteren Seite mit einer der Anzahl der Schaftrippen (19) entsprechenden Zahl von auf die Geometrie der Schaftrippen (19) abgestimmten, bevorzugt jeweils als Meißel oder Raspel ausgebildeten Bearbeitungsstegen (31) versehen ist.

18. Implantatsystem nach Anspruch 17,
**dadurch gekennzeichnet,**
**dass** jeder Bearbeitungssteg (31) weniger hoch ist als die entsprechende Schaftrippe (19).

19. Implantatsystem nach Anspruch 17 oder 18,
**dadurch gekennzeichnet,**
**dass** alle Bearbeitungsstege (31) zumindest im Wesentlichen die gleiche Länge aufweisen.

20. Implantatsystem nach einem der vorhergehenden Ansprüche,
**dadurch gekennzeichnet,**
**dass** das erste Instrument (13) als Raspel ausgebildet ist.

21. Implantatsystem nach einem der vorhergehenden Ansprüche,
**dadurch gekennzeichnet,**
**dass** das zweite Instrument (15) als Meißel oder Raspel ausgebildet ist.

22. Implantatsystem nach einem der vorhergehenden Ansprüche,
**dadurch gekennzeichnet,**
**dass** das erste Instrument (13) und der Führungskörper (14) für das zweite Instrument (15) separate Teile sind.

23. Implantatsystem nach einem der vorhergehenden Ansprüche,
**dadurch gekennzeichnet,**
**dass** das erste Instrument (13) gleichzeitig als Führungskörper (14) für das zweite Instrument (15) ausgebildet ist.

## Claims

1. An implant system having
- at least one shaft (11) of a prosthesis, in particular a hip joint prosthesis, which can be anchored without cement; and
- a set of instruments (13, 14, 15) for the preparation of the bone in which the shaft (11) is to be anchored,
wherein the shaft (11) has an arcuate base body (17) with a trapezoidal or rectangular cross-section
**characterized in that** the set of instruments includes a first instrument (13) which is shaped in accordance with the arcuate shape of the shaft (11) and with which a recess serving for the reception of the base body (17) can be established in the bone; and
**in that** the shaft (11) is provided with ribs (19) in the proximal region and the set of instruments includes a guide body (14) shaped in accordance with the arcuate shape of the shaft (11) for a second instrument (15) with which structures can be manufactured in the bone serving to fix a path for the shaft ribs (19) when the guide body (14) is located in the recess.

2. An implant system in accordance with claim 1, **characterized in that** the trapezoidal or rectangular cross-section of the shaft (11) has pronounced edges in its longitudinal direction.

3. An implant system in accordance with claim 1 or claim 2, **characterized in that** the arcuate shape is selected in particular with respect to curvature and length such that, for oppositely disposed edges, an end position of the base body (17) results which is clearly defined by a plurality of contact positions (21), in particular three contact positions, at the cortex of the bone.

4. An implant system in accordance with any one of the preceding claims, **characterized in that** the extent of the shaft ribs (19) is adapted to the arcuate shape such that, for oppositely disposed edges, an end position of the base body (17) can be reached which is clearly defined by a plurality of contact positions (21), in particular three contact positions, at the cortex of the bone.

5. An implant system in accordance with any one of the preceding claims, **characterized in that** at least some shaft ribs (19) are straight at least regionally.

6. An implant system in accordance with any one of the preceding claims, **characterized in that** at least some shaft ribs (19) extend in a curved manner at least regionally, preferably with a constant radius of curvature.

7. An implant system in accordance with any one of the preceding claims, **characterized in that** the shaft ribs (19) extend toward distal starting from a common plane (23), with the common plane (23) preferably being disposed in the region of a resection plane of the bone with the shaft (11) inserted.

8. An implant system in accordance with any one of the preceding claims, **characterized in that** the shaft ribs (19) are of different length.

9. An implant system in accordance with any one of the preceding claims, **characterized in that** straight shaft ribs (19) and tangents at curved shaft ribs (19) extend toward the shaft axis (A) at the same angle (α) different from zero.

10. An implant system in accordance with any one of the preceding claims, **characterized in that** the distal end of the first instrument (13) projects beyond a base body (17') of the first instrument (13) corresponding to the base body (17) of the shaft (11).

11. An implant system in accordance with any one of the preceding claims, **characterized in that** the shaft (11), the first instrument (13) and the guide body (14) are each provided with a flattened area (25) at the lateral side in the region of the distal end, with the distal tip being asymmetrically displaced toward medial.

12. An implant system in accordance with any one of the preceding claims, **characterized in that** the first instrument (13) is provided with a respective chamfer (26) at the anterior and posterior sides in the distal end region.

13. An implant system in accordance with any one of the preceding claims, **characterized in that** the second instrument (15) is movable toward distal along a guide path pre-determined by the guide body (14).

14. An implant system in accordance with claim 13, **characterized in that** the guide path is curved in accordance with shaft ribs (19) extending in a curved manner.

15. An implant system in accordance with any one of the preceding claims, **characterized in that** the second instrument (15) is guided in a compulsory manner at least regionally at the guide body (14).

16. An implant system in accordance with any one of the preceding claims, **characterized in that** the second instrument (15) is U-shaped or fork-shaped and can be pushed onto the guide body (14) with the free ends of the U arms or fork arms (29) at the front.

17. An implant system in accordance with any one of the preceding claims, **characterized in that** the second instrument (15) is provided at its front side and/or rear side with a number of working webs (31) corresponding to the number of shaft ribs (19), matched to the geometry of the shaft ribs (19) and preferably made as a chisel or a rasp.

18. An implant system in accordance with claim 17, **characterized in that** each working web (31) is less high than the corresponding shaft rib (19).

19. An implant system in accordance with claim 17 or claim 18, **characterized in that** all working webs (31) have at least substantially the same length.

20. An implant system in accordance with any one of the preceding claims, **characterized in that** the first instrument (13) is made as a rasp.

21. An implant system in accordance with any one of the preceding claims, **characterized in that** the second instrument (15) is made as a chisel or as a rasp.

22. An implant system in accordance with any one of the preceding claims, **characterized in that** the first instrument (13) and the guide body (14) for the second instrument (15) are separate parts.

23. An implant system in accordance with any one of the preceding claims, **characterized in that** the first instrument (13) is simultaneously made as a guide body (14) for the second instrument (15).

## Revendications

1. Système d'implant, comportant
- au moins une tige (11), susceptible d'être ancrée sans ciment, d'une prothèse, en particulier d'une prothèse de la hanche, et
- un jeu d'instruments (13, 14, 15) pour préparer l'os dans lequel la tige (11) doit être ancrée,
la tige (11) présentant un corps de base (17) de forme arquée avec section transversale trapézoïdale ou rectangulaire, **caractérisé en ce que** le jeu d'instruments comprend un premier instrument formé selon la forme arquée de la tige (11), avec lequel un creux servant à recevoir le corps de base (17) peut être réalisé dans l'os, et **en ce que** la tige (11) est pourvue de nervures (19) dans la région proximale, et le jeu d'instrument comprend un corps de guidage (14) formé selon la forme arquée de la tige (11), pour un deuxième instrument (15) par lequel des structures peuvent être réalisées dans l'os lorsque le corps de guidage (14) se trouve dans le creux pour déterminer une voie pour les nervures de tige (19).

2. Système d'implant selon la revendication 1,
**caractérisé en ce que**
la tige (11) présente, avec sa section transversale en forme trapézoïdale ou rectangulaire, des arêtes saillantes dans sa direction longitudinale.

3. Système d'implant selon la revendication 1 ou 2,
**caractérisé en ce que**
la forme arquée est choisie telle du point de vue courbure et longueur qu'il en résulte pour des arêtes opposées une position définitive du corps de base (17) définie de manière univoque par plusieurs, en particulier par trois points d'appui (21) sur la corticale de l'os.

4. Système d'implant selon l'une des revendications précédentes,
**caractérisé en ce que**
le tracé des nervures de tige (19) est adapté à la forme arquée de telle sorte que pour des arêtes opposées, on peut atteindre une position définitive du corps de base (17), définie de manière univoque par plusieurs, en particulier par trois points d'appui (21) sur la corticale de l'os.

5. Système d'implant selon l'une des revendications précédentes,
**caractérisé en ce que**
au moins quelques nervures de tige (19) sont réalisées rectilignes au moins partiellement.

6. Système d'implant selon l'une des revendications précédentes,
**caractérisé en ce que**
au moins quelques nervures de tige (19) s'étendent curvilignes au moins partiellement, de préférence avec un rayon de courbure constant.

7. Système d'implant selon l'une des revendications précédentes,
**caractérisé en ce que**
les nervures de tige (19) s'étendent depuis un plan (23) commun vers le côté distal, et de préférence, lorsque la tige (11) est mise en place, le plan (23) commun est situé dans la région d'un plan de résection de l'os.

8. Système d'implant selon l'une des revendications précédentes,
**caractérisé en ce que**
les nervures de tige (19) sont de différentes longueurs.

9. Système d'implant selon l'une des revendications précédentes,
**caractérisé en ce que**
des nervures de tige (19) rectilignes et des tangentes sur des nervures de tige (19) curvilignes s'étendent sous le même angle (α) différent de zéro par rapport à l'axe de tige (A).

10. Système d'implant selon l'une des revendications précédentes,
**caractérisé en ce que**
l'extrémité distale du premier instrument (13) fait saillie en direction distale au-delà d'un premier corps de base (17') du premier instrument (13), qui correspond au corps de base (17) de la tige (11).

11. Système d'implant selon l'une des revendications précédentes,
**caractérisé en ce que**
la tige (11), le premier instrument (13) et le corps de guidage (14) sont pourvus chacun d'un méplat (25) sur la face latérale dans la région de l'extrémité distale, la pointe distale étant déplacée asymétriquement vers le côté médial.

12. Système d'implant selon l'une des revendications précédentes,
**caractérisé en ce que**
le premier instrument (13) est pourvu d'un chanfrein (26) respectif dans la région d'extrémité distale sur la face antérieure et sur la face postérieure.

13. Système d'implant selon l'une des revendications précédentes,
**caractérisé en ce que**
le deuxième instrument (15) peut être déplacé vers le côté distal le long d'une voie de guidage prédéterminée par le corps de guidage (14).

14. Système d'implant selon la revendication 13
**caractérisé en ce que**
la voie de guidage est curviligne selon des nervures de tige (19) s'étendant avec une courbure correspondante.

15. Système d'implant selon l'une des revendications précédentes,
**caractérisé en ce que**
le deuxième instrument (15) est guidé par force au moins partiellement sur le corps de guidage (14).

16. Système d'implant selon l'une des revendications précédentes,
**caractérisé en ce que**
le deuxième instrument (15) est réalisé en forme de U ou en forme de fourche et peut être enfilé sur le corps de guidage (14) avec les extrémités libres des bras en forme de U ou de fourche (29) en avant.

17. Système d'implant selon l'une des revendications précédentes,
**caractérisé en ce que**
sur sa face antérieure et/ou sur sa face postérieure, le deuxième instrument (15) est pourvu d'un certain nombre, correspondant au nombre de nervures de tige (19), de barrettes d'usinage adaptées à la géométrie des nervures de tige (19) et réalisées de préférence sous forme de burin ou de râpe.

18. Système d'implant selon la revendication 17,
**caractérisé en ce que**
chaque barrette d'usinage (31) est moins haute que la nervure de tige (19) correspondante.

19. Système d'implant selon la revendication 17 ou 18,
**caractérisé en ce que**
toutes les barrettes d'usinage (31) présentent au moins sensiblement la même longueur.

20. Système d'implant selon l'une des revendications précédentes,
**caractérisé en ce que**
le premier instrument (13) est réalisé sous forme de râpe.

21. Système d'implant selon l'une des revendications précédentes,
**caractérisé en ce que**
le deuxième instrument (15) est réalisé sous forme de burin ou de râpe.

22. Système d'implant selon l'une des revendications précédentes,
**caractérisé en ce que**
le premier instrument (13) et le corps de guidage (14) pour le deuxième instrument (15) sont des pièces séparées.

23. Système d'implant selon l'une des revendications précédentes,
**caractérisé en ce que**
le premier instrument (13) est réalisé en même temps sous forme de corps de guidage (14) pour le deuxième instrument (15).
